# EUROPÄISCHE PATENTSCHRIFT

(11) **EP 2 830 710 B1**
(45) Veröffentlichungstag und Bekanntmachung des Hinweises auf die Patenterteilung: **17.02.2021**
(21) Anmeldenummer: 13730149.5
(22) Anmeldetag: 07.06.2013
(51) Int. Cl.: A61N 5/10

(54) **VERFAHREN UND VORRICHTUNG ZUM BESTIMMEN EINES BESTRAHLUNGSPLANS FÜR EINE PARTIKELBESTRAHLUNGSANLAGE**
METHOD AND DEVICE FOR DETERMINING AN IRRADIATION PLAN FOR A PARTICLE IRRADIATION UNIT
PROCÉDÉ ET DISPOSITIF DE DÉTERMINATION D'UN PLAN DE RAYONNEMENT POUR UNE INSTALLATION DE RAYONNEMENT PARTICULAIRE

(30) Priorität: 13.07.2012 DE 102012212341
(43) Veröffentlichungstag der Anmeldung: 04.02.2015
(73) Patentinhaber: Siemens Healthcare GmbH, 91052 Erlangen (DE)
(72) Erfinder: BOHSUNG, Jörg, 68161 Mannheim (DE); ELSÄSSER, Thilo, 91054 Erlangen (DE); GRÖZINGER, Sven Oliver, 91353 Hausen (DE); KAWRAKOW, Iwan, 1336 Ljulin (BG); KIM, Johann, 91301 Forchheim (DE); NEUHAUSER, Robert, 85375 Neufahrn (DE); RIETZEL, Eike, 64331 Weiterstadt (DE); THILMANN, Oliver, 86159 Augsburg (DE)
(86) Internationale Anmeldenummer: PCT/EP2013/061834
(87) Internationale Veröffentlichungsnummer: WO 2014/009073

(56) Entgegenhaltungen:
- DE-A1-102008 009 765
- DE-A1-102009 010 284
- DE-A1-102009 043 548

## Beschreibung

Die vorliegende Erfindung betrifft ein Verfahren und eine Vorrichtung zur Bestimmung eines Bestrahlungsplans für eine Partikelbestrahlungsanlage und eine entsprechend ausgestaltete Partikelbestrahlungsanlage.

Beispielsweise im Rahmen einer Therapieplanung bei der Partikeltherapie wird im Vorfeld ein Bestrahlungsplan erstellt, welcher Steuerparameter zur Bestrahlung eines Untersuchungsobjekts definiert. Mit dem Bestrahlungsplan wird die Bestrahlung eines Gegenstands gemäß bestimmten Vorgaben (z. B. Zielvolumen oder Dosisverteilung) geplant.

Die Partikeltherapie ist ein mittlerweile etabliertes Verfahren, mit welchem insbesondere von Tumorerkrankungen befallenes Gewebe bestrahlt wird. Bei der Partikeltherapie werden geladene Partikel, wie beispielsweise Elektronen, Protonen oder Kohlenstoff-Ionen oder andere Ionen oder auch Photonen oder Neutronen auf hohe Energien beschleunigt, zu einem Partikelstrahl geformt und über ein Hochenergie-Transportsystem zu einem (oder mehreren) Bestrahlungsräumen geführt. In einem Bestrahlungsraum wird das Zielvolumen des Behandlungsobjekts mit dem Partikelstrahl bestrahlt, wobei zwangsweise auch Gewebe außerhalb des Zielvolumens bestrahlt wird.

Wenn bei der Partikeltherapie Beschleuniger mit einer aktiven Energievariation eingesetzt werden, werden für die Bestrahlung des Zielvolumens Teilchenstrahlen unterschiedlicher Energie eingesetzt, wodurch sich so genannte Isoenergieschichten ausbilden, welche innerhalb und außerhalb des Zielvolumens liegen können. In einer Isoenergieschicht werden von dem Teilchenstrahl Teilchen derselben Energie appliziert, so dass die Teilchen des Teilchenstrahls zur Einstellung auf die jeweilige Isoenergieschicht jeweils eine Energie aufweisen, welche sich von der Energie der Teilchen für andere Isoenergieschichten unterscheidet. Dabei werden nach Möglichkeit alle Teilchen einer Isoenergieschicht mit Hilfe eines Spills (d.h. mittels einer einzigen Beschleunigerfüllung) appliziert, da das Laden bzw. Erzeugen eines neuen Spills mehrere Sekunden dauert. Die Unterbrechungszeit zur Erzeugung eines neuen Spills macht insgesamt abhängig von der zu applizierenden Teilchenzahl einen signifikanten Anteil der Gesamtbestrahlungsdauer aus. Um eine möglichst kurze patientenfreundliche Bestrahlung, eine genaue Applikation der Dosis im Zielvolumen sowie einen möglichst wirtschaftlichen Betrieb der Partikelbestrahlungsanlage zu gewährleisten, muss die direkte Bestrahlungszeit (d.h. die Zeit, in der der Patient dem Teilchenstrahl tatsächlich ausgesetzt ist) sowie die Gesamtbestrahlungsdauer (d.h. die Zeit, die der Patient im Bestrahlungsraum verbringt) so kurz wie möglich gehalten werden. Dabei muss jedoch die Qualität des Bestrahlungsplans beachtet werden, welche letztlich vom behandelnden Arzt zu beurteilen ist.

Die Druckschrift DE 102008009765 A1 offenbart eine Steuerung zum Steuern einer Anlage zum Bestrahlen eines Zielvolumens mittels eines Scanverfahrens. Ferner offenbart die Druckschrift DE 102009043548 A1 ein Verfahren zur Anpassung und Optimierung einer Bestrahlungsplanung. Zudem offenbart die Druckschrift DE 102009010284 A1 ein Verfahren zur Bestimmung von Steuerparametern für eine Partikelbestrahlungsanlage, wobei zur Bestimmung der Steuerparameter eine Partikelanzahlverteilung ermittelt wird.

Daher stellt sich die vorliegende Erfindung die Aufgabe, die Gesamtbestrahlungsdauer unter Beibehaltung einer akzeptablen Planqualität bei der Bestimmung eines Bestrahlungsplans für eine Partikelbestrahlungsanlage möglichst kurz zu halten.

Erfindungsgemäß wird diese Aufgabe durch ein Verfahren zum Bestimmen eines Bestrahlungsplans für eine Partikelbestrahlungsanlage nach Anspruch 1, durch eine Vorrichtung zum Bestimmen eines Bestrahlungsplans für eine Partikelbestrahlungsanlage nach Anspruch 8, durch eine Partikelbestrahlungsanlage nach Anspruch 10, durch ein Computerprogrammprodukt nach Anspruch 11 oder durch einen elektronisch lesbaren Datenträger nach Anspruch 12 gelöst. Die abhängigen Ansprüche definieren bevorzugte und vorteilhafte Ausführungsformen der vorliegenden Erfindung.

Im Rahmen der vorliegenden Erfindung wird ein Verfahren zum (insbesondere automatischen) Bestimmen eines Bestrahlungsplans für eine Partikelbestrahlungsanlage bereitgestellt. Dabei wird mit Hilfe der Partikelbestrahlungsanlage gemäß dem Bestrahlungsplan ein Zielvolumen innerhalb eines Untersuchungsobjekts mit einem Partikelstrahl bzw. Teilchenstrahl bestrahlt. Der Bestrahlungsplan wird dabei ausgehend von einem vorgegebenen Zielvolumen und einer vorbestimmten Dosisverteilung (Soll-Dosisverteilung) innerhalb dieses Zielvolumens bestimmt, um anhand des Bestrahlungsplans die Dosis des Teilchenstrahls mit einer hohen Qualität (z. B. möglichst exakt entsprechend der Soll-Dosisverteilung) in dem Zielvolumen, welches mehrere Isoenergieschichten umfasst, zu deponieren oder zu applizieren. Dabei wird eine Randbedingung, welche von dem Bestrahlungsplan einzuhalten ist, für eine oder mehrere der Isoenergieschichten vorgegeben. Diese Randbedingung umfasst dabei eine oder mehrere der im Folgenden aufgelisteten Bedingungen.

### 1. Minimale Grenzenergie.

Eine zu bestrahlende Isoenergieschicht muss zumindest diese minimale Grenzenergie aufweisen. Anders ausgedrückt werden diejenigen Isoenergieschichten nicht bestrahlt (von dem Bestrahlungsplan nicht berücksichtigt), zu deren Bestrahlung der Teilchenstrahl eine Energie aufweisen würde, welche unterhalb der minimalen Grenzenergie liegt.

Die Bedingung bezüglich der minimalen Grenzenergie kann dabei absolut (d.h. ohne Berücksichtigung der jeweiligen Energie der Isoenergieschichten) oder relativ (d.h. unter Berücksichtigung der jeweiligen Energie der Isoenergieschichten) definiert werden. Wenn beispielsweise die 3 (n) Isoenergieschichten mit der niedrigsten Energie nicht berücksichtigt bzw. bestrahlt werden sollen, wird die minimale Grenzenergie relativ zu der Energie der Isoenergieschicht mit der drittniedrigsten (n-niedrigsten) Energie definiert und liegt z. B. leicht oberhalb der Energie (der Teilchen) der Isoenergieschicht mit der drittniedrigsten (n-niedrigsten) Energie. Dagegen berücksichtigt eine absolute Definition der minimalen Grenzenergie die jeweilige Energie der Isoenergieschichten nicht, so dass beispielsweise alle Isoenergieschichten unterhalb einer vorbestimmten Energie (z.B. 150 MeV) nicht bestrahlt werden, wobei dieses Kriterium unabhängig davon ist, wie viele oder welcher Prozentsatz der Isoenergieschichten dann nicht bestrahlt werden.

### 2. Maximale Grenzenergie.

Zur Bestrahlung einer Isoenergieschicht darf keine Energie benötigt werden, welche oberhalb der maximalen Grenzenergie liegt. Anders ausgedrückt werden diejenigen Isoenergieschichten nicht bestrahlt (von dem Bestrahlungsplan nicht berücksichtigt), zu deren Bestrahlung der Teilchenstrahl eine Energie aufweisen würde, welche oberhalb der maximalen Grenzenergie liegt.

Die maximale Grenzenergie kann dabei absolut (d.h. ohne Berücksichtigung der jeweiligen Energie der Isoenergieschichten) oder relativ (d.h. unter Berücksichtigung der jeweiligen Energie der Isoenergieschichten) definiert werden. Wenn beispielsweise die 3 (n) Isoenergieschichten mit der höchsten Energie nicht berücksichtigt bzw. bestrahlt werden sollen, wird die maximale Grenzenergie relativ zu der Energie der Isoenergieschicht mit der dritthöchsten (n-höchsten) Energie definiert und liegt leicht unterhalb der Energie (der Teilchen) der Isoenergieschicht mit der dritthöchsten (n-höchsten) Energie. Dagegen berücksichtigt eine absolute Definition der maximalen Grenzenergie die jeweilige Energie der Isoenergieschichten nicht.

### 3. Minimale Rasterpunktanzahl.

Es werden gemäß dieser Bedingung nur Isoenergieschichten bestrahlt, deren Rasterpunktanzahl nicht unterhalb der minimalen Rasterpunktanzahl liegt.

Unter einem Rasterpunkt wird dabei ein Punkt in einer zum Teilchenstrahl orthogonalen Ebene durch das Isozentrum verstanden. Der Teilchenstrahl wird jeweils auf einen Rasterpunkt gerichtet, welcher somit die Richtung des Teilchenstrahls bestimmt.

Wenn die Anzahl der Rasterpunkte für eine Isoenergieschicht sehr klein ist, wird die Planqualität des Bestrahlungsplans durch die Bestrahlung dieser Isoenergieschicht nicht signifikant verbessert. Durch diese Bedingung wird die Bestrahlung einer solchen Isoenergieschicht vermieden, wodurch die Bestrahlungszeit eines Patienten vorteilhafterweise verringert werden kann.

### 4. Minimale Gesamtpartikelanzahl.

Es werden gemäß dieser Bedingung nur Isoenergieschichten bestrahlt, deren Gesamtpartikelanzahl nicht unterhalb der minimalen Gesamtpartikelanzahl liegt.

### 5. Minimale Gesamtdosis.

Es werden gemäß dieser Bedingung nur Isoenergieschichten bestrahlt, für welche die gesamte in der jeweiligen Isoenergieschicht applizierte Dosis zumindest nicht kleiner als die minimale Gesamtdosis ist.

### 6. Minimaler Dosisbeitrag einer zu applizierenden Gesamtdosis.

Es werden gemäß dieser Bedingung nur Isoenergieschichten bestrahlt, für welche die in der jeweiligen Isoenergieschicht applizierte Dosis zumindest den minimalen Dosisbeitrag zu der im Zielvolumen insgesamt (für alle Isoenergieschichten) zu applizierenden Gesamtdosis aufweist. Dieser minimale Dosisbeitrag kann dabei ein bestimmter Prozentsatz der im Zielvolumen zu applizierenden Gesamtdosis sein. Dann werden nur diejenigen Isoenergieschichten bestrahlt, bei denen das Verhältnis der in der jeweiligen Isoenergieschicht applizierten Dosis zur im Zielvolumen zu applizierenden Gesamtdosis zumindest diesem Prozentsatz entspricht.

Bei einer Bestrahlungsplanung nach dem Stand der Technik kann es bei der Optimierung anhand von Planqualitätskriterien vorkommen, dass die für eine Isoenergieschicht optimierte Gesamtteilchenzahl so niedrig ist, dass die in der Isoenergieschicht applizierte Gesamtdosis vernachlässigbar gegenüber der Gesamtdosis für den vollständigen Bestrahlungsplan ist oder zumindest einen für die Planqualität vernachlässigbar kleinen Beitrag liefert. Durch diese Bedingung wird vermieden, dass eine solche Isoenergieschicht bestrahlt wird, wodurch die Gesamtbestrahlungszeit um 3 bis 5 s (typische Zeit zur Bestrahlung einer Isoenergieschicht) verringert werden kann.

### 7. Minimaler Beitrag zu einer Zielfunktion, welche zur Bestimmung des Bestrahlungsplans berechnet wird.

Für jede Isoenergieschicht kann der Beitrag zu der Zielfunktion bestimmt werden. Gemäß dieser Bedingung werden nur Isoenergieschichten bestrahlt, für welche der Beitrag zu der Zielfunktion zumindest dem minimalen Beitrag entspricht.

### 8. Minimaler Dosiskompensationsfehler.

Der Dosiskompensationsfehler gibt das Ausmaß eines Fehlers an, welcher durch das Nicht-Bestrahlen einer bestimmten Isoenergieschicht trotz entsprechender Kompensation durch die bestrahlten Isoenergieschichten auftritt. Gemäß dieser Bedingung werden nur Isoenergieschichten bestrahlt, für welche der Dosiskompensationsfehler nicht kleiner als der minimale Dosiskompensationsfehler ist.

Damit der erfindungsgemäß bestimmte Bestrahlungsplan die Randbedingung bzw. eine oder mehrere der vorab genannten Bedingungen einhält, werden die entsprechenden Bedingungen insbesondere bereits bei der Erstellung des Bestrahlungsplans berücksichtigt. D.h. es wird bereits bei der Erstellung des Bestrahlungsplans darauf geachtet, dass keine Isoenergieschicht die Randbedingung bzw. eine oder mehrere der Bedingungen verletzt.

Es ist allerdings erfindungsgemäß auch möglich, den Bestrahlungsplan in einem ersten Schritt ohne Berücksichtigung der Randbedingung zu erstellen und dann in einem zweiten Schritt alle Isoenergieschichten von der Bestrahlung auszunehmen, welche die vorgegebene Randbedingung nicht erfüllen. Anschließend ist es optional möglich, den Bestrahlungsplan unter Ausschluss der ausgenommenen Isoenergieschichten nachzuoptimieren.

Zur Bestimmung des Bestrahlungsplans wird die Zielfunktion optimiert, wobei jede (bestrahlte) Isoenergieschicht einen bestimmten Beitrag zu dieser Zielfunktion liefert. Der Beitrag der jeweiligen Isoenergieschicht kann sich dabei aus einer Summe aus die Zielfunktion begünstigenden Beiträgen (z. B. Dosisbeitrag zur Gesamtdosis) und aus die Zielfunktion verschlechternden Beiträgen (Zeitdauer der Bestrahlung, Bestrahlung gesunden Gewebes) zusammensetzen. Bei der Überprüfung, ob eine Isoenergieschicht zumindest den minimalen Beitrag zu der Zielfunktion beisteuert, wird somit nur ein begünstigender Beitrag der jeweiligen Isoenergieschicht berücksichtigt. D.h. eine Isoenergieschicht, welche (insgesamt) nur einen die Zielfunktion verschlechternden Beitrag liefert, hat die Bedingung nicht erfüllt.

Erfindungsgemäß ist es dabei möglich, dass für jede Isoenergieschicht eine individuelle Randbedingung vorgegeben wird, so dass jede Isoenergieschicht eine oder mehrere individuell auf die jeweilige Isoenergieschicht zugeschnittene Bedingungen einhalten muss.

Es ist allerdings auch möglich, dass eine oder mehrere der vorab gelisteten Bedingungen für jede Isoenergieschicht gelten, so dass in diesem Fall für alle Isoenergieschichten quasi dieselbe(n) Bedingung(en) gilt/gelten.

Die Bewertung des erfindungsgemäß erstellten Bestrahlungsplans kann durch eines der folgenden Vorgehen vorgenommen werden.

Gemäß einer ersten Ausführungsform zur Bewertung des Bestrahlungsplans kann ein weiterer Bestrahlungsplan erfindungsgemäß bestimmt werden, wobei zur Bestimmung dieses weiteren Bestrahlungsplans für eine oder für mehrere derjenigen Isoenergieschichten, bei welchen gemäß dem zu bewertenden Bestrahlungsplan keine Bestrahlung stattfindet, da die Randbedingung verletzt ist, die Randbedingung aufgehoben wird. Dadurch erfolgt die Bestimmung des weiteren Bestrahlungsplans, ohne eine Einschränkung durch die Randbedingung für die eine oder die mehreren Isoenergieschichten zu berücksichtigen. Mit anderen Worten kann die eine oder können die mehreren Isoenergieschichten, welche gemäß dem zu bewertenden Bestrahlungsplan nicht bestrahlt werden, bei dem weiteren Bestrahlungsplan zur Bestrahlung vorgesehen werden, ohne dass dabei die Randbedingung zu berücksichtigen ist. Zur Bewertung des Bestrahlungsplans wird zum einen eine Planqualität des Bestrahlungsplans erstellt, welche im Wesentlichen abhängig von der erzielten Dosisverteilung ist. Diese Planqualität wird mit einer weiteren Planqualität verglichen, welche für den weiteren Bestrahlungsplan erstellt wird, wobei auch diese weitere Planqualität im Wesentlichen von der von dem weiteren Bestrahlungsplan erzielten Dosisverteilung abhängt. Im Normalfall ist die Bewertung des Bestrahlungsplans umso besser anzusehen, je geringer der Unterschied zwischen der Planqualität und der weiteren Planqualität ist.

Im Extremfall kann bei dieser ersten Ausführungsform der weitere Bestrahlungsplan ohne jegliche Berücksichtigung einer Randbedingung bezüglich irgendeiner Isoenergieschicht erstellt werden.

Anders ausgedrückt wird zur Bewertung des erfindungsgemäß erstellten Bestrahlungsplans gemäß der ersten Ausführungsform überprüft, wie stark sich die Planqualität des Bestrahlungsplans dadurch verschlechtert, dass die Randbedingung einzuhalten ist.

Gemäß einer zweiten erfindungsgemäßen Ausführungsform zur Bewertung des erfindungsgemäß erstellten Bestrahlungsplans kann eine Dosisverteilung bestimmt werden, welche gemäß dem zu bewertenden Bestrahlungsplan appliziert wird. Diese Dosisverteilung wird nun mit der vorbestimmten Dosisverteilung verglichen, wobei das Ergebnis dieses Vergleichs der Bewertung des Bestrahlungsplans entspricht.

Je mehr die für den erfindungsgemäß erstellten Bestrahlungsplan bestimmte Dosisverteilung der vorbestimmten Dosisverteilung entspricht, desto besser sollte die Bewertung des erfindungsgemäß erstellten Bestrahlungsplans ausfallen.

Gemäß einer dritten erfindungsgemäßen Ausführungsform zur Bewertung des erfindungsgemäß erstellten Bestrahlungsplans werden ein oder mehrere Planungsergebnisse verwendet, welche für den Bestrahlungsplan erstellt werden. Dabei wird das Planungsergebnis oder werden die Planungsergebnisse ausgewählt aus folgender Gruppe von Planungsergebnissen:
- Ein Dosis-Volumen-Histogramm, welches für den zu bewertenden Bestrahlungsplan erstellt wird.
- Eine Angabe, wie viel der Dosis laut dem zu bewertenden Bestrahlungsplan im Zielvolumen appliziert wird. Dabei sollte die laut Bestrahlungsplan im Zielvolumen applizierte Dosis der vorgegebenen Dosisverteilung bzw. Soll-Dosisverteilung möglichst gut entsprechen. Dieses Planungsergebnis beinhaltet auch eine Angabe über die Einhaltung von Toleranzgrenzen hinsichtlich der Dosisverteilung innerhalb des Zielvolumens ("dose constraints of planning target volume").
- Eine Angabe, wie viel der Dosis laut dem zu bewertenden Bestrahlungsplan in mindestens einem Risikoorgan des Untersuchungsobjekts appliziert wird. Aus dieser Angabe lässt sich beispielsweise die Information, ob bestimmte Toleranzgrenzen bezüglich der Risikoorgane eingehalten werden, ableiten.

Diese Bewertung anhand eines oder mehrerer der vorab aufgeführten Planungsergebnisse kann auch zur Bestimmung der Planqualität des weiteren Bestrahlungsplans eingesetzt werden.

Die Zielfunktion, welche zur Bestimmung des Bestrahlungsplans berechnet wird, kann einen Bestrafungsterm umfassen, wobei dieser Bestrafungsterm die Zielfunktion umso mehr verschlechtert, je kleiner ein Abstand gemäß dem Bestrahlungsplan zu der jeweiligen Bedingung ist.

Dieser Sachverhalt soll im Folgenden nochmals genauer erläutert werden.

Zur Bestimmung des Bestrahlungsplans wird gemäß einer bevorzugten Ausführungsform mittels eines Optimierungsalgorithmus ein optimaler Funktionswert der Zielfunktion bestimmt. Dabei berücksichtigt die Zielfunktion zum einen den optimalen Funktionswert begünstigende Beiträge bzw. Terme, welche z.B. ein Optimum annehmen, wenn die gemäß dem Bestrahlungsplan applizierte Dosis der Soll-Dosis entspricht. Durch Berücksichtigung des Bestrafungsterms wird der Funktionswert der Zielfunktion und damit die Bewertung des zu erstellenden Bestrahlungsplans umso schlechter, je näher der Bestrahlungsplan bezüglich einer oder mehrerer Isoenergieschichten an durch die einzuhaltende Bedingung definierte Grenze bzw. Schwelle rückt. Der Bestrafungsterm bildet demnach ein Regulativ, um den Bestrahlungsplan derart zu erstellen, dass die vorgegebenen Bedingungen eingehalten werden. Mit anderen Worten wird die vordefinierte Randbedingung bereits in Form des Bestrafungsterms bei dem Optimierungsalgorithmus, mit welchem die Zielfunktion optimiert wird, berücksichtigt.

Zur Vorgabe der Randbedingung für die eine oder für die mehreren Isoenergieschichten kann wie folgt vorgegangen werden.

Es wird ein vorläufiger Bestrahlungsplan bestimmt, um anhand dieses Bestrahlungsplans den Teilchenstrahl gemäß der Soll-Dosisverteilung in dem Zielvolumen zu applizieren. Dabei wird dieser vorläufige Bestrahlungsplan insbesondere ohne Berücksichtigung der Randbedingung erstellt. Die Randbedingung, welche dann bei der Erstellung des eigentlichen Bestrahlungsplans einzuhalten ist, wird ausgehend von dem vorläufigen Bestrahlungsplan erstellt.

Indem zur Erstellung der Randbedingung der vorläufige Bestrahlungsplan erstellt wird, kann die Randbedingung (z.B. die minimale Rasterpunktanzahl, die minimale Gesamtpartikelanzahl, die minimale Gesamtdosis, der minimale Beitrag zur Zielfunktion, der minimale Dosiskompensationsfehler) derart bestimmt werden, dass beispielsweise eine vorbestimmte Anzahl (z.B. 10 %) der Isoenergieschichten, welche laut dem vorläufigen Bestrahlungsplan zu bestrahlen sind, die Randbedingung bzw. eine der Bedingungen verletzen.

Im Rahmen der vorliegenden Erfindung wird auch eine Vorrichtung zum Bestimmen eines Bestrahlungsplans für eine Partikelbestrahlungsanlage bereitgestellt. Die Partikelbestrahlungsanlage bestrahlt dabei abhängig von dem bestimmten Bestrahlungsplan ein Zielvolumen innerhalb eines Untersuchungsobjekts mit einem Teilchenstrahl oder Partikelstrahl. Die Vorrichtung umfasst Eingabemittel, Rechenmittel und Ausgabemittel. Mittels der Eingabemittel werden der Vorrichtung das Zielvolumen, eine vorbestimmte Dosisverteilung (Soll-Dosisverteilung) und eine Randbedingung für eine oder mehrere der Isoenergieschichten vorgegeben. Die Rechenmittel bestimmen den Bestrahlungsplan derart, dass die Teilchen des Teilchenstrahls entsprechend der vorbestimmten Dosisverteilung in dem Zielvolumen, welches mehrere Isoenergieschichten umfasst, abgegeben werden. Der Bestrahlungsplan wird mit den Ausgabemitteln ausgegeben. Die Rechenmittel der erfindungsgemäßen Vorrichtung bestimmen den Bestrahlungsplan zusätzlich derart, dass die Randbedingung für die eine oder für die mehreren Isoenergieschichten eingehalten wird. Die Randbedingung umfasst dabei mindestens eine derjenigen Bedingungen, welche bezüglich des erfindungsgemäßen Verfahrens vorab aufgelistet worden sind.

Die Vorteile der erfindungsgemäßen Vorrichtung entsprechen im Wesentlichen den Vorteilen des erfindungsgemäßen Verfahrens, welche vorab im Detail ausgeführt sind, so dass hier auf eine Wiederholung verzichtet wird.

Darüber hinaus stellt die vorliegende Erfindung eine Partikelbestrahlungsanlage mit einer erfindungsgemäßen Vorrichtung bereit.

Des Weiteren beschreibt die vorliegende Erfindung ein Computerprogrammprodukt, insbesondere eine Software, welche man in einen Speicher einer programmierbaren Steuereinrichtung bzw. Rechenmittel einer Partikelbestrahlungsanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechenden Rechenmittel bzw. Steuereinrichtung zu laden ist.

Schließlich offenbart die vorliegende Erfindung einen elektronisch lesbaren Datenträger, z.B. eine DVD, ein Magnetband oder einen USB-Stick, auf welchem elektronisch lesbare Steuerinformationen, insbesondere Software (vgl. oben), gespeichert ist. Wenn diese Steuerinformationen (Software) von dem Datenträger gelesen und in Steuermittel bzw. eine Recheneinheit einer Partikelbestrahlungsanlage gespeichert werden, können alle erfindungsgemäßen Ausführungsformen des vorab beschriebenen Verfahrens durchgeführt werden.

Die Auswahl der einzuhaltenden Bedingungen oder die genaue Bestimmung bzw. Definition der einzelnen Bedingung können dabei interaktiv oder mittels einer Konfigurationseinstellung von einem Benutzer vorgegeben werden.

Durch die Vorgabe und Einhaltung der Randbedingung kann die Anzahl der zu bestrahlenden Isoenergieschichten im Vergleich zum Stand der Technik verringert werden. Dadurch kann die Gesamtbestrahlungsdauer deutlich verkürzt werden, was zum größeren Komfort für den Patienten und zu einer Steigerung der Effektivität der Partikelbestrahlungsanlage führt. Die Einführung der vorliegenden Erfindung erfordert vorteilhafterweise keinen Eingriff in die Strahlenapplikation oder in den Beschleuniger selbst, da es sich um eine programmtechnische Ausgestaltung handelt. Durch eine Wählbarkeit der Bedingungen und eine Anzeige der Auswirkungen auf die Qualität des Bestrahlungsplans kann darüber hinaus vorteilhafterweise fallbezogen ein optimaler Kompromiss zwischen einer verkürzten Gesamtbestrahlungsdauer und der Qualität des Bestrahlungsplans gefunden werden.

Die vorliegende Erfindung ist insbesondere zur Erhöhung des Patientendurchsatzes bei der Partikeltherapie geeignet. Selbstverständlich ist die vorliegende Erfindung nicht auf diesen bevorzugten Anwendungsbereich eingeschränkt, da die vorliegende Erfindung prinzipiell überall dort eingesetzt werden kann, wo mit Teilchen oder Partikeln Energie bzw. eine Dosis in einem Zielvolumen appliziert wird.

Im Folgenden wird die vorliegende Erfindung anhand erfindungsgemäßer Ausführungsformen mit Bezug zu den Figuren im Detail beschrieben.

Fig. 1 stellt schematisch einen Überblick über den Aufbau einer Partikelbestrahlungsanlage dar.

In Fig. 2 ist schematisch dargestellt, wie ein Zielvolumen mittels einer Partikelbestrahlungsanlage bestrahlt wird.

Fig. 3 zeigt einen Ablaufplan der vorliegenden Erfindung.

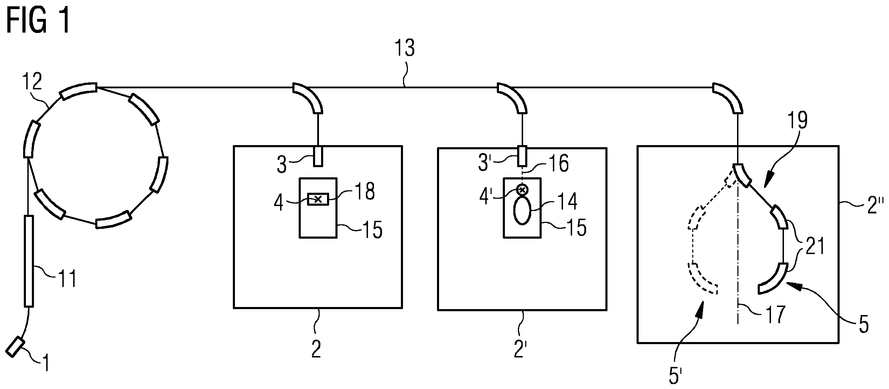

Die in Fig. 1 schematisch dargestellte Partikelbestrahlungsanlage 20 bestrahlt einen auf einer Positionierungsvorrichtung 15 (einem Tisch) liegenden Patienten 14 (siehe Bestrahlungsraum 2') mit einem Partikel bzw. Teilchen 16 umfassenden Strahl, welcher im Folgenden als Partikelstrahl bzw. Teilchenstrahl 16 bezeichnet wird. Mit einem solchen Teilchenstrahl 16 kann beispielsweise ein Tumor des Patienten 14 mit hochenergetischen Partikeln bestrahlt werden. Es ist allerdings auch möglich, die Partikelbestrahlungsanlage 20 zur Bestrahlung eines nicht-lebenden Objektes 18 einzusetzen, wie es im Bestrahlungsraum 2 am Beispiel eines Wasserphantoms 18 dargestellt ist.

Als Partikel bzw. Teilchen werden beispielsweise Protonen, Pionen, Heliumionen, Kohlenstoffionen, aber auch Ionen anderer Elemente eingesetzt. Dazu werden die entsprechenden Partikel in einer Partikelquelle bzw. Ionenquelle 1 erzeugt und in einem Vorbeschleuniger 11 (z. B. einem Linearbeschleuniger) auf ein erstes Energieniveau beschleunigt. Anschließend werden die Teilchen in einem Ringbeschleuniger 12 (z. B. einem Synchrotron oder Zyklotron) auf eine zur Bestrahlung benötigte Energie beschleunigt. Der aus dem Ringbeschleuniger 12 austretende Teilchenstrahl wird von einem Hochenergiestrahl-Transportsystem 13 in einen oder mehrere Bestrahlungsräume 2, 2', 2'' transportiert, und dort zur Bestrahlung eines Zielvolumens eines Patienten 14 eingesetzt. Die Bestrahlung erfolgt von einer festen Richtung aus, so dass der zu bestrahlende Körper 14, 18 vorher raumfest mittels der Positionierungsvorrichtung 15 in dem Bestrahlungsraum 2, 2' angeordnet wird. Die Bestrahlungsräume 2, 2' werden daher auch als so genannte "fixed beam"-Räume bezeichnet. Dagegen existiert im Bestrahlungsraum 2'' eine um eine Achse 17 beweglich angeordnete, insbesondere drehbare Gantry 19, mittels welcher der zu bestrahlende Körper von verschiedenen Richtungen aus (mit verschiedenen Feldern) bestrahlt werden kann. Dazu wird der Teilchenstrahl 16 mit Hilfe einer Strahlführung 21 der Gantry 19 entsprechend auf den zu bestrahlenden Körper gerichtet. In Fig. 1 sind zwei Positionen 5, 5' dargestellt, obwohl mehrere Positionen möglich sind.

In den Bestrahlungsräumen 2, 2' tritt der Teilchenstrahl 16 aus einem Strahlauslass 3, 3' aus und trifft auf den Körper 14 bzw. 18, in welchem sich das zu bestrahlende Zielvolumen befindet. Das Zielvolumen liegt dabei normalerweise in dem Isozentrum 4, 4' des jeweiligen Bestrahlungsraums 2, 2'.

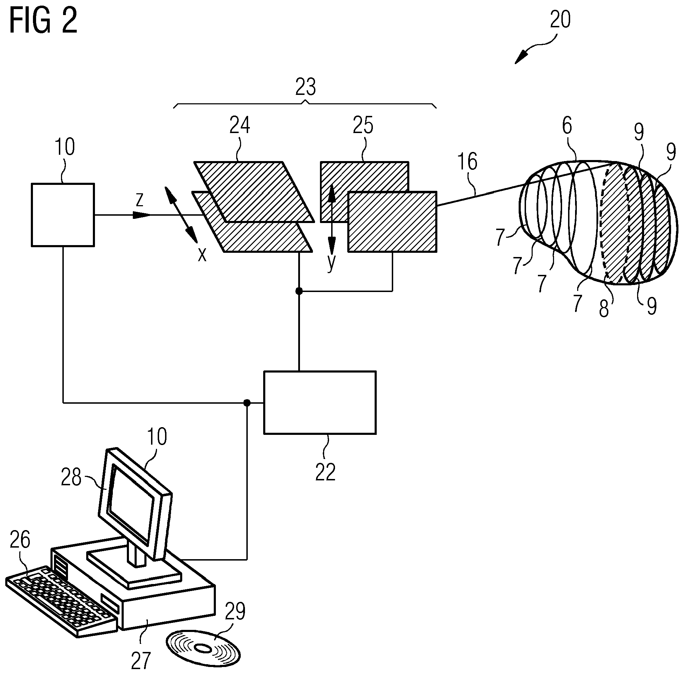

In Fig. 2 ist schematisch ein Zielvolumen 6 dargestellt, welches von einem mittels einer Partikelbestrahlungsanlage 20 erzeugten Teilchenstrahl 16 bestrahlt wird. Die Partikelbestrahlungsanlage 20 umfasst neben einer Bestrahlungsplanungsvorrichtung 10 eine Strahlerzeugungsvorrichtung 30, eine Rasterscan-Einrichtung 23 und eine Steuerung 22 für die Rasterscan-Einrichtung 23. Die Rasterscan-Einrichtung 23 weist wiederum eine erste Partikelablenkung 24 und eine zweite Partikelablenkung 25 auf, welche jeweils insbesondere Magnete umfassen. Mit Hilfe der beiden Partikelablenkungen 24, 25 kann der Teilchenstrahl 16 sowohl horizontal als auch vertikal abgelenkt werden, was durch die zueinander senkrecht stehenden Pfeile x, y dargestellt ist. Daher ist die Rasterscan-Einrichtung 23 in der Lage, den Teilchenstrahl 16 auf einen beliebigen Punkt (xᵢ, yᵢ) einer Fläche innerhalb der x-y-Ebene zu lenken. Jeder dieser Punkte wird zusammen mit der jeweils eingesetzten Partikelenergie als Scan Spot, Rasterpunkt oder Abtastpunkt bezeichnet. Demnach ist ein Rasterpunkt zum einen durch die Ausrichtung des Teilchenstrahls 16 (x- bzw. y-Richtung) und zum anderen durch seine Partikelenergie bestimmt. Mit anderen Worten existieren für bestimmte x- und y-Koordinaten mehrere Rasterpunkte mit unterschiedlichen Partikelenergien. Die Partikelenergie bestimmt dabei quasi (unter Berücksichtigung des durchstrahlten Körpers, welcher z.B. durch eine CT-Aufnahme ermittelt wird) die Koordinate in der z-Richtung (senkrecht auf der x- bzw. y-Achse), wobei im Allgemeinen gilt, dass die z-Position des Bragg-Peaks umso weiter in Richtung des Teilchenstrahls 16 innerhalb des Zielvolumens 6 liegt, umso größer die Partikelenergie ist. Da allerdings die Eindringtiefe vom Gewebe bzw. Material abhängig ist, welches der Teilchenstrahl 16 durchläuft, gilt obiger Zusammenhang nur für dieselben x-bzw. y-Positionen exakt.

Unter dem Bragg-Peak wird dabei derjenige Punkt bzw. Bereich verstanden, in welchem der Teilchenstrahl entlang seiner Bahn den größten Anteil seiner Dosis appliziert.

Das mit dem Teilchenstrahl 16 zu bestrahlende Zielvolumen 6 wird dabei in Form von Isoenergieschichten 7-9 bestrahlt. In den Rasterpunkten derselben Isoenergieschicht 7-9 werden dabei jeweils Partikel mit derselben Energie appliziert. Unter der Voraussetzung, dass der Teilchenstrahl 16 auf seinem Weg zu der entsprechenden Isoenergieschicht 7-9 ein homogenes Volumen durchläuft, liegen die Isoenergieschichten 7-9 senkrecht zur z-Achse, wie es vereinfachend in Fig. 2 dargestellt ist.

Zur Einstellung des Teilchenstrahls 16 auf eine entsprechende Isoenergieschicht 7-9 (d.h. zur Positionierung des Bragg-Peaks auf einer Isoenergieschicht 7-9) wird den Partikeln des Teilchenstrahls 16 jeweils eine entsprechende Anfangsenergie zugewiesen, indem die Partikel auf eine dieser Anfangsenergie entsprechende Geschwindigkeit beschleunigt werden. Die Anfangsenergie beschreibt dabei die Energie eines Partikels, welches dieses Partikel vor dem Auftreffen auf das Objekt 14 bzw. 18 aufweist. Zur Bestrahlung derjenigen Isoenergieschicht 7, welche dem Strahlauslass 3, 3' am nächsten liegt (am weitesten links in Fig. 2), werden dazu Partikel mit der niedrigsten Energie eingesetzt, wohingegen zur Bestrahlung derjenigen Isoenergieschicht 9, welche von dem Strahlauslass 3, 3' am weitesten entfernt angeordnet ist (am weitesten rechts in Fig. 2), die Partikel mit der höchsten Energie verwendet werden.

Zur Bestrahlung des gesamten Zielvolumens 6 werden die Isoenergieschichten 7-9 nacheinander bestrahlt, wobei in der Regel mit der Isoenergieschicht 9, welche am weitesten vom Strahlauslass 3, 3' entfernt ist, begonnen wird und dann mit der jeweils benachbarten Isoenergieschicht fortgefahren wird. Um bestimmte Rasterpunkte innerhalb derselben Isoenergieschicht 7-9 mit unterschiedlichen Energien zu bestrahlen, wird insbesondere die Zeitspanne variiert, während welcher der entsprechende Rasterpunkt von dem Teilchenstrahl 16 bestrahlt wird. Je länger der entsprechende Rasterpunkt von dem Teilchenstrahl 16 bestrahlt wird, desto mehr Energie (höhere Dosis) wird in dem entsprechenden Rasterpunkt deponiert.

Bei dem in Fig. 2 dargestellten Zielvolumen 6 wird aktuell die Isoenergieschicht 8 von dem Teilchenstrahl 16 bestrahlt, während die drei Isoenergieschichten 9 bereits bestrahlt worden sind und die weiter links (in Fig. 2) liegenden vier Isoenergieschichten 7 noch auf ihre Bestrahlung warten. Bevor das Zielvolumen 6 bestrahlt wird, wird ein Bestrahlungsplan erstellt, mittels welchem das Scannen bzw. Abtasten des Zielvolumens 6 mit dem Teilchenstrahl 16 erfolgt. Der Bestrahlungsplan bestimmt dabei insbesondere Steuerparameter zur Steuerung der Partikelbestrahlungsanlage 20. Die Erstellung des Bestrahlungsplans wird dabei mit Hilfe einer Bestrahlungsplanungsvorrichtung 10 (beispielsweise einem PC) durchgeführt.

Zur Durchführung der eigentlichen Bestrahlung wird der Bestrahlungsplan von der Bestrahlungsplanungsvorrichtung 10 an die Strahlerzeugungsvorrichtung 30 und die Steuerung 22 der Rasterscan-Einrichtung 23 weitergeleitet. In Fig. 2 ist die Bestrahlungsplanungsvorrichtung 10 quasi als Bestandteil der Partikelbestrahlungsanlage 20 dargestellt. Natürlich ist es ebenso gut erfindungsgemäß möglich, dass der von der Bestrahlungsplanungsvorrichtung 10 erstellte Bestrahlungsplan auf einen Datenträger 29 geladen wird, über welchen dann der Bestrahlungsplan in die Partikelbestrahlungsanlage 20 geladen wird. In diesem Fall müssen die Bestrahlungsplanungsvorrichtung 10 und die Partikelbestrahlungsanlage 20 nicht miteinander kommunikationstechnisch verbunden sein. Darüber hinaus kann zwischen der Erstellung des Bestrahlungsplans und der anhand des Bestrahlungsplans durchgeführten Bestrahlung ein gewisser Zeitraum, beispielsweise mehrere Tage, liegen.

Zur Erstellung des Bestrahlungsplans benötigt die Bestrahlungsplanungsvorrichtung 10 die Lage und die Ausmaße des zu bestrahlenden Zielvolumens 6 (z. B. eines zu bestrahlenden Tumors). Darüber hinaus wird bei der Bestrahlung eines Patienten 14 die Beschaffenheit des Gewebes benötigt, welches von dem Teilchenstrahl 16 auf dem Weg zu dem Zielvolumen 6 durchstrahlt wird. Diese Informationen können beispielsweise mittels eines Computer- oder Magnetresonanztomographen ermittelt werden und dann über entsprechende Eingabemittel 26 an die Bestrahlungsplanungsvorrichtung 10 übermittelt werden. Die Bestrahlungsplanungsvorrichtung 10 bestimmt mit Hilfe ihrer Rechenmittel 27 ausgehend von diesen Informationen und einer vorbestimmten Dosisverteilung (Soll-Dosisverteilung) den Bestrahlungsplan. Der Bestrahlungsplan gibt dabei insbesondere an, wie viele Partikel einer bestimmten Energie an einem Rasterpunkt zu applizieren sind.

Ein Patient muss während der Bestrahlung fixiert werden, um eine Bewegung des Zielvolumens 6 möglichst auszuschließen. Aus diesem Grund sollte die Bestrahlungsdauer möglichst kurz gehalten werden. Darüber hinaus ermöglicht eine kurze Bestrahlungsdauer vorteilhafterweise einen höheren Patientendurchsatz. Auf der anderen Seite sollte die Dosisverteilung gemäß Bestrahlungsplan möglichst gut der Soll-Dosisverteilung entsprechen. Indem erfindungsgemäß die Anzahl der bestrahlten Isoenergieschichten im Vergleich zum Stand der Technik kleiner ist, da nur Isoenergieschichten bestrahlt werden, welche die Randbedingung einhalten, weist ein erfindungsgemäß erstellter Bestrahlungsplan eine geringere Gesamtbestrahlungsdauer auf.

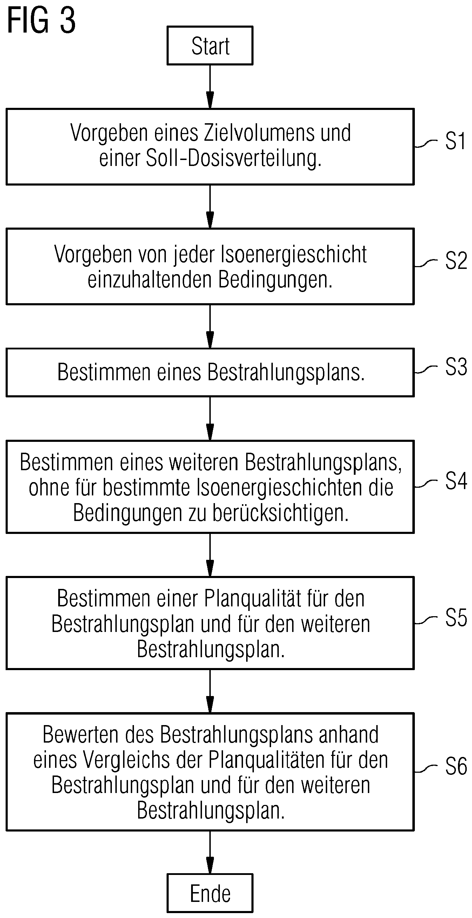

In Fig. 3 ist ein Ablaufplan der vorliegenden Erfindung dargestellt.

In einem ersten Schritt S1 wird das Zielvolumen und die Soll-Dosisverteilung vorgegeben, während in einem zweiten Schritt S2 die von jeder Isoenergieschicht einzuhaltenden Bedingungen (beispielsweise minimale Rasterpunktanzahl, minimale Gesamtpartikelanzahl, minimale Gesamtdosis) vorgegeben werden. Ausgehend von diesen Vorgaben wird im Schritt S3 ein Bestrahlungsplan bestimmt.

Zur Bewertung dieses in Schritt S3 bestimmten Bestrahlungsplans wird im Schritt S4 ein weiterer Bestrahlungsplan erstellt. Bei der Erstellung dieses weiteren Bestrahlungsplans werden für bestimmte derjenigen Isoenergieschichten, welche laut dem im Schritt S3 bestimmten Bestrahlungsplans nicht bestrahlt werden, da sie den vorgegebenen Bedingungen nicht genügen, diese Bedingungen ausgesetzt. Mit anderen Worten werden bei der Erstellung des weiteren Bestrahlungsplans die vorgegebenen Bedingungen bezüglich der bestimmten Isoenergieschichten nicht überprüft, so dass der weitere Bestrahlungsplan mit einem größeren Freiheitsgrad erstellt wird.

Anschließend werden im Schritt S5 sowohl für den Bestrahlungsplan als auch für den weiteren Bestrahlungsplan jeweils eine Planqualität bestimmt, welche insbesondere eine Aussage darüber trifft, inwieweit die von dem jeweiligen Bestrahlungsplan geplante Dosisverteilung der Soll-Dosisverteilung entspricht. Anhand eines Vergleichs der Planqualität des Bestrahlungsplans und der Planqualität des weiteren Bestrahlungsplans wird der Bestrahlungsplan im Schritt S6 bewertet.

Durch diesen Vergleich der Planqualitäten kann beispielsweise die Veränderung der dreidimensionalen Dosisverteilung begutachtet werden, wenn laut dem Bestrahlungsplan bestimmte (oder alle) zur Entfernung markierten Isoenergieschichten beispielsweise über eine Benutzerschnittstelle interaktiv wieder zugeschaltet werden, indem das Einhalten der Bedingungen für diese Isoenergieschichten ausgesetzt wird. Um die Entscheidung zu erleichtern, welche Isoenergieschichten wieder zugeschaltet werden, kann die Zeitersparnis angezeigt werden, welche bei einem Nicht-Bestrahlen der jeweiligen Isoenergieschicht eingespart wird. Der Vergleich der Planqualitäten des Bestrahlungsplans und des weiteren Bestrahlungsplans kann dabei durch eine Anzeige bestimmter Planungsergebnisse (z.B. Dosis-Volumen-Histogramme) unterstützt werden, um dem Benutzer eine weitere Entscheidungshilfe zu geben.

## Patentansprüche

1. Computerimplementiertes Verfahren zum Bestimmen eines Bestrahlungsplans für eine Partikelbestrahlungsanlage (20),
wobei mit der Partikelbestrahlungsanlage (20) abhängig von dem Bestrahlungsplan ein Zielvolumen (6) innerhalb eines Untersuchungsobjekts (14; 18) mit einem Teilchenstrahl (16) bestrahlt werden kann,
wobei das Zielvolumen (6) und eine vorbestimmte Dosisverteilung vorgegeben werden,
wobei der Bestrahlungsplan bestimmt wird, um den Teilchenstrahl (16) gemäß der vorbestimmten Dosisverteilung in dem Zielvolumen (6), welches mehrere Isoenergieschichten (7-9) umfasst, applizieren zu können,
wobei eine Randbedingung für mindestens eine der Isoenergieschichten (7-9) vorgegeben wird,
**dadurch gekennzeichnet,**
**dass** der Bestrahlungsplan zusätzlich derart bestimmt wird, dass die Randbedingung für die mindestens eine Isoenergieschicht eingehalten wird und nur Isoenergieschichten bestrahlt werden, welche die jeweils vorgegebene Randbedingung erfüllen, und dass die Randbedingung zumindest eine von folgenden Bedingungen umfasst:
• eine minimale Grenzenergie,
• eine minimale Rasterpunktanzahl,
• eine minimale Gesamtpartikelanzahl,
• eine minimale Gesamtdosis, und
• ein minimaler Dosiskompensationsfehler, wobei der Dosiskompensationsfehler einen Fehler angibt, welcher durch das Nicht-Bestrahlen der jeweiligen Isoenergieschicht trotz entsprechender Kompensation durch die bestrahlten Isoenergieschichten auftritt.

2. Verfahren nach Anspruch 1,
**dadurch gekennzeichnet,**
**dass** für jede Isoenergieschicht eine individuelle Randbedingung vorgegeben wird.

3. Verfahren nach Anspruch 1,
**dadurch gekennzeichnet,**
**dass** für jede Isoenergieschicht bezüglich zumindest einer der Bedingungen dieselbe Bedingung vorgegeben wird.

4. Verfahren nach einem der vorgehenden Ansprüche,
**dadurch gekennzeichnet,**
**dass** ein weiterer Bestrahlungsplan gemäß dem Verfahren nach einem der vorhergehenden Ansprüche bestimmt wird,
**dass** zur Bestimmung des weiteren Bestrahlungsplans für mindestens eine bestimmte der Isoenergieschichten, bei welcher gemäß dem Bestrahlungsplan keine Teilchen appliziert werden, da die Randbedingung verletzt ist, die Randbedingung aufgehoben wird, so dass die Bestimmung des weiteren Bestrahlungsplans erfolgt, ohne dass eine Restriktion durch die Randbedingung bezüglich der mindestens einen bestimmten Isoenergieschicht existiert, und
**dass** zur Bewertung des Bestrahlungsplans eine Planqualität des Bestrahlungsplans abhängig von der Dosisverteilung mit einer weiteren Planqualität des weiteren Bestrahlungsplans abhängig von der Dosisverteilung verglichen wird.

5. Verfahren nach einem der vorhergehenden Ansprüche,
**dadurch gekennzeichnet,**
**dass** eine Dosisverteilung, welche gemäß dem Bestrahlungsplan appliziert wird, bestimmt wird, und
**dass** zur Bewertung des Bestrahlungsplans diese Dosisverteilung mit der vorbestimmten Dosisverteilung verglichen wird.

6. Verfahren nach Anspruch 4 oder 5,
**dadurch gekennzeichnet,**
**dass** zur Bewertung des Bestrahlungsplans zumindest ein Planungsergebnis folgender Gruppe von Planungsergebnissen für den Bestrahlungsplan und für den weiteren Bestrahlungsplan bestimmt wird, wobei die Gruppe der Planungsergebnisse umfasst:
• ein Dosis-Volumen-Histogramm,
• eine Angabe, wie viel der Dosis laut Bestrahlungsplan im Zielvolumen appliziert wird, und
• eine Angabe, wie viel der Dosis laut Bestrahlungsplan in mindestens einem Risikoorgan des Untersuchungsobjekts appliziert wird.

7. Verfahren nach einem der vorgehenden Ansprüche,
**dadurch gekennzeichnet,**
**dass** die Zielfunktion, welche zur Bestimmung des Bestrahlungsplans berechnet wird, einen Bestrafungsterm umfasst, und dass der Bestrafungsterm einen zu optimierenden Funktionswert der Zielfunktion umso mehr verschlechtert, je geringer ein Abstand gemäß dem Bestrahlungsplan zu der jeweiligen Bedingung ist.

8. Verfahren nach einem der vorgehenden Ansprüche,
**dadurch gekennzeichnet,**
**dass** ein vorläufiger Bestrahlungsplan bestimmt wird, um den Teilchenstrahl (16) gemäß der vorbestimmten Dosisverteilung in dem Zielvolumen (6) applizieren zu können, und dass die Randbedingung abhängig von dem vorläufigen Bestrahlungsplan vorgegeben wird.

9. Vorrichtung zum Bestimmen eines Bestrahlungsplans für eine Partikelbestrahlungsanlage (20),
wobei mit der Partikelbestrahlungsanlage (20) abhängig von dem Bestrahlungsplan ein Zielvolumen (6) innerhalb eines Untersuchungsobjekts (14; 18) mit einem Teilchenstrahl (16) bestrahlt wird,
wobei die Vorrichtung (10) Eingabemittel (26), Rechenmittel (27) und Ausgabemittel (28) umfasst,
wobei der Vorrichtung (10) mittels der Eingabemittel (26) das Zielvolumen (6) und eine vorbestimmte Dosisverteilung vorgebbar sind,
wobei die Rechenmittel (27) den Bestrahlungsplan bestimmen, um den Teilchenstrahl (16) gemäß der vorbestimmten Dosisverteilung in dem Zielvolumen (6), welches mehrere Isoenergieschichten (7-9) umfasst, zu applizieren, und
wobei die Ausgabemittel (28) den Bestrahlungsplan ausgeben, wobei der Vorrichtung (10) mittels der Eingabemittel (26) eine Randbedingung für mindestens eine der Isoenergieschichten (7-9) vorgebbar ist,
**dadurch gekennzeichnet,**
**dass** die Rechenmittel (27) den Bestrahlungsplan zusätzlich derart bestimmen, dass die Randbedingung für die mindestens eine Isoenergieschicht (7-9) eingehalten wird und nur Isoenergieschichten bestrahlt werden, welche die jeweils vorgegebene Randbedingung erfüllen, und
**dass** die Randbedingung zumindest eine von folgenden Bedingungen umfasst:
• eine minimale Grenzenergie,
• eine minimale Rasterpunktanzahl,
• eine minimale Gesamtpartikelanzahl,
• eine minimale Gesamtdosis, und
• ein minimaler Dosiskompensationsfehler, wobei der Dosiskompensationsfehler einen Fehler angibt, welcher durch das Nicht-Bestrahlen der jeweiligen Isoenergieschicht trotz entsprechender Kompensation durch die bestrahlten Isoenergieschichten auftritt.

10. Vorrichtung nach Anspruch 9,
**dadurch gekennzeichnet,**
**dass** die Vorrichtung (10) zur Durchführung des Verfahrens nach einem der Ansprüche 1-8 ausgestaltet ist.

11. Partikelbestrahlungsanlage mit einer Vorrichtung (10) nach Anspruch 9 oder 10.

12. Computerprogrammprodukt, welches ein Programm umfasst und direkt in einen Speicher einer programmierbaren Steuereinrichtung einer Partikelbestrahlungsanlage (20) ladbar ist, mit Programm-Mitteln, um alle Schritte des Verfahrens nach einem der Ansprüche 1-8 auszuführen, wenn das Programm in der Steuereinrichtung der Partikelbestrahlungsanlage (20) ausgeführt wird.

13. Elektronisch lesbarer Datenträger mit darauf gespeicherten elektronisch lesbaren Steuerinformationen, welche derart ausgestaltet sind, dass sie bei Verwendung des Datenträgers (29) in einer Steuereinrichtung einer Partikelbestrahlungsanlage (20) das Verfahren nach einem der Ansprüche 1-8 durchführen.

## Claims

1. Computer-implemented method for determining an irradiation plan for a particle irradiation unit (20),
wherein the particle irradiation unit (20) can be used to irradiate a target volume (6) within an examination object (14; 18) with a particle beam (16) depending on the irradiation plan,
wherein the target volume (6) and a predetermined dose distribution are prescribed,
wherein the irradiation plan is determined in order to be able to apply the particle beam (16) in accordance with the predetermined dose distribution in the target volume (6) which comprises a plurality of isoenergy layers (7-9),
wherein a boundary condition is prescribed for at least one of the isoenergy layers (7-9),
**characterized**
**in that** the irradiation plan is additionally determined in such a way that the boundary condition for the at least one isoenergy layer is observed and only isoenergy layers which satisfy the respectively prescribed boundary layer are irradiated, and
**in that** the boundary condition comprises at least one of the following conditions:
• a minimum peak energy,
• a minimum number of raster points,
• a minimum overall number of particles,
• a minimum overall dose, and
• a minimum dose compensation error, wherein the dose compensation error specifies an error which occurs as a result of the non-irradiation of the respective isoenergy layer despite corresponding compensation by the irradiated isoenergy layers.

2. Method according to Claim 1,
**characterized**
**in that** an individual boundary condition is prescribed for each isoenergy layer.

3. Method according to Claim 1,
**characterized**
**in that** the same condition is prescribed for each isoenergy layer in respect of at least one of the conditions.

4. Method according to any one of the preceding claims,
**characterized**
**in that** a further irradiation plan is determined in accordance with the method according to any one of the preceding claims, in that, for determining the further irradiation plan, the boundary condition is lifted for at least one specific one of the isoenergy layers, in which no particles are applied in accordance with the irradiation plan since the boundary condition is broken, such that the further irradiation plan is determined without there being a restriction by the boundary condition in respect of the at least one specific isoenergy layer, and
**in that** a plan quality of the irradiation plan depending on the dose distribution is compared to a further plan quality of the further irradiation plan depending on the dose distribution for evaluating the irradiation plan.

5. Method according to any one of the preceding claims,
**characterized**
**in that** a dose distribution, which is applied in accordance with the irradiation plan, is determined and
**in that** this dose distribution is compared to the predetermined dose distribution for evaluating the irradiation plan.

6. Method according to Claim 4 or 5,
**characterized**
**in that** at least one planning result of the following group of planning results for the irradiation plan and for the further irradiation plan is determined for evaluating the irradiation plan, wherein the group of planning results comprises:
• a dose/volume histogram,
• a specification of how much of the dose is applied in the target volume according to the irradiation plan, and
• a specification of how much of the dose is applied in at least one organ at risk of the examination object according to the irradiation plan.

7. Method according to any one of the preceding claims,
**characterized**
**in that** the target function, which is calculated for determining the irradiation plan, comprises a penalty term and in that the penalty term reduces the quality of a function value to be optimized of the target function further as a distance in accordance with the irradiation plan to the respective condition decreases.

8. Method according to any one of the preceding claims,
**characterized**
**in that** a preliminary irradiation plan is determined in order to be able to apply the particle beam (16) in the target volume (6) in accordance with the predetermined dose distribution and in that the boundary condition is prescribed depending on the preliminary irradiation plan.

9. Device for determining an irradiation plan for a particle irradiation unit (20),
wherein the particle irradiation unit (20) is used to irradiate a target volume (6) within an examination object (14; 18) with a particle beam (16) depending on the irradiation plan, wherein the device (10) comprises input means (26), computing means (27) and output means (28),
wherein the target volume (6) and a predetermined dose distribution are prescribable for the device (10) by means of the input means (26),
wherein the computing means (27) determine the irradiation plan in order to apply the particle beam (16) in the target volume (6) which comprises a plurality of isoenergy layers (7-9), in accordance with the predetermined dose distribution, and wherein the output means (28) output the irradiation plan, wherein a boundary condition for at least one of the isoenergy layers (7-9) is prescribable for the device (10) by means of the input means (26),
**characterized**
**in that** the computing means (27) additionally determine the irradiation plan in such a way that the boundary condition for the at least one isoenergy layer (7-9) is observed and only isoenergy layers which satisfy the respectively prescribed boundary layer are irradiated, and
**in that** the boundary condition comprises at least one of the following conditions:
• a minimum peak energy,
• a minimum number of raster points,
• a minimum overall number of particles,
• a minimum overall dose, and
• a minimum dose compensation error, wherein the dose compensation error specifies an error which occurs as a result of the non-irradiation of the respective isoenergy layer despite corresponding compensation by the irradiated isoenergy layers.

10. Device according to Claim 9,
**characterized**
**in that** the device (10) is embodied for performing the method according to any one of Claims 1-8.

11. Particle irradiation unit comprising a device (10) according to Claim 9 or 10.

12. Computer program product, which comprises a program and is directly loadable into a memory of a programmable control apparatus of a particle irradiation unit (20), comprising program means for executing all steps of the method according to any one of Claims 1-8 when the program is executed in the control apparatus of the particle irradiation unit (20).

13. Electronically readable data medium with electronically readable control information stored thereon which is configured in such a way that, when the data medium (29) is used in a control apparatus of a particle irradiation unit (20), it performs the method according to any one of Claims 1-8.

## Revendications

1. Procédé mis en œuvre par ordinateur de détermination d'un plan d'exposition à un rayonnement pour une installation ( 20 ) d'exposition à un rayonnement particulaire,
dans lequel, par l'installation ( 20 ) d'exposition à un rayonnement particulaire, on peut, en fonction du plan d'exposition à un rayonnement, exposer à un faisceau ( 16 ) de particules un volume ( 6 ) cible au sein d'un objet ( 14 ; 18 ) à examiner,
dans lequel on prescrit le volume ( 6 ) cible et une répartition de dose déterminée à l'avance,
dans lequel on définit le plan d'exposition à un rayonnement, afin de pouvoir appliquer le faisceau ( 16 ) de particules suivant la répartition de dose déterminée à l'avance au volume ( 6 ) cible, qui comprend plusieurs couches ( 7 à 9 ) d'isoénergie,
dans lequel on prescrit une condition aux limites pour au moins l'une des couches ( 7 à 9 ) d'iso-énergie,
**caractérisé**
**en ce que** l'on définit, en outre, le plan d'exposition à un rayonnement, de manière à respecter la condition aux limites pour la au moins une couche d'iso-énergie et on n'expose à un rayonnement que des couches d'iso-énergie, qui satisfont la condition aux limites prescrite, respectivement, et
**en ce que** la condition aux limites comprend au moins l'une des conditions aux limites suivantes :
• une énergie limite minimum,
• un nombre de points de trame minimum,
• un nombre total de particules minimum,
• une dose totale minimum, et
• un manque de compensation de dose minimum, le manque de compensation de dose indiquant un manque, qui se produit par la non soumission à un rayonnement de la couche d'iso-énergie respective, en dépit d'une compensation correspondante par les couches d'iso-énergie exposées au rayonnement.

2. Procédé suivant la revendication 1,
**caractérisé**
**en ce que** l'on prescrit, pour chaque couche d'iso-énergie, une condition aux limites individuelle.

3. Procédé suivant la revendication 1,
**caractérisé**
**en ce que** l'on prescrit, pour chaque couche d'iso-énergie, en ce qui concerne au moins l'une des conditions, la même condition.

4. Procédé suivant l'une des revendications précédentes,
**caractérisé**
**en ce que** l'on définit un autre plan d'exposition à un rayonnement suivant le procédé selon l'une des revendications précédentes,
**en ce que**, pour définir l'autre plan d'exposition à un rayonnement pour au moins une déterminée des couches d'isoénergie, pour laquelle, suivant le plan d'exposition à un rayonnement, il n'est pas appliqué de particule, car la condition aux limites est enfreinte, on supprime la condition aux limites de manière à effectuer la définition de l'autre plan d'exposition au rayonnement sans qu'existe une restriction par la condition aux limites en ce qui concerne la au moins une couche d'iso-énergie déterminée, et
**en ce que**, pour évaluer le plan d'exposition à un rayonnement, on compare une qualité du plan d'exposition à un rayonnement en fonction de la répartition de dose à une autre qualité de l'autre plan d'exposition à un rayonnement en fonction de la répartition de dose.

5. Procédé suivant l'une des revendications précédentes,
**caractérisé**
**en ce que** l'on détermine une répartition de dose, qui est appliquée suivant le plan d'exposition à un rayonnement, et
**en ce que**, pour évaluer le plan d'exposition à un rayonnement, on compare cette répartition de dose à la répartition de dose déterminée à l'avance.

6. Procédé suivant la revendication 4 ou 5,
**caractérisé**
**en ce que**, pour évaluer le plan d'exposition à un rayonnement, on détermine au moins un résultat du plan du groupe suivant de résultats du plan pour le plan d'exposition à un rayonnement et pour l'autre plan d'exposition à un rayonnement, le groupe des résultats du plan comprenant :
• un histogramme dose-volume,
• une indication de combien la dose suivant le plan d'exposition à un rayonnement est appliquée au volume cible et
• une indication de combien la dose suivant le plan d'exposition à un rayonnement est appliquée à au moins un organe à risque de l'objet à examiner.

7. Procédé suivant l'une des revendications précédentes,
**caractérisé**
**en ce que** la fonction cible, que l'on calcule pour la détermination du plan d'exposition à un rayonnement, comprend un terme de pénalité et en ce que le terme de pénalité rend d'autant moins bonne une valeur à optimiser de la fonction cible, qu'est plus petite une distance suivant le plan d'exposition à un rayonnement à la condition respective.

8. Procédé suivant l'une des revendications précédentes,
**caractérisé**
**en ce que** l'on définit un plan d'exposition à un rayonnement provisoire, afin de pouvoir appliquer le faisceau ( 16 ) de particules suivant la répartition de dose déterminée à l'avance au volume ( 6 ) cible, et
**en ce que** l'on prescrit la condition aux limites en fonction du plan d'exposition à un rayonnement provisoire.

9. Système de détermination d'un plan d'exposition à un rayonnement pour une installation ( 20 ) d'exposition à un rayonnement particulaire,
dans lequel, par l'installation ( 20 ) d'exposition à un rayonnement particulaire, on peut, en fonction du plan d'exposition à un rayonnement, exposer à un faisceau ( 16 ) de particules un volume ( 6 ) cible au sein d'un objet ( 14 ; 18 ) à examiner,
dans lequel le système ( 10 ) comprend des moyens ( 26 ) d'entrée, des moyens ( 27 ) de calcul et des moyens ( 28 ) de sortie,
dans lequel il peut être prescrit au système ( 10 ), à l'aide des moyens ( 26 ) d'entrée, le volume ( 6 ) cible et une répartition de dose déterminée à l'avance,
dans lequel les moyens ( 27 ) de calcul déterminent le plan d'exposition à un rayonnement pour appliquer le faisceau ( 16 ) de particules, suivant la répartition de dose déterminée à l'avance, au volume ( 6 ) cible, qui comprend plusieurs couches ( 7 à 9 ) d'iso-énergie, et
dans lequel les moyens ( 28 ) de sortie émettent le plan d'exposition à un rayonnement,
dans lequel il peut être prescrit au système ( 10 ), à l'aide des moyens ( 26 ) d'entrée, une condition aux limites pour au moins l'une des couches ( 7 à 9 ) d'iso-énergie,
**caractérisé**
**en ce que** les moyens ( 27 ) de calcul définissent, en outre, le plan d'exposition à un rayonnement de manière à respecter la condition aux limites pour la au moins une couche ( 7 à 9 ) d'iso-énergie et ne sont soumis à un rayonnement que des couches d'iso-énergie, qui satisfont la condition aux limites respective prescrite, et
**en ce que** la condition aux limites comprend au moins l'une des conditions suivantes :
• une énergie limite minimum,
• un nombre de points de trame minimum,
• un nombre total de particules minimum,
• une dose totale minimum, et
• un manque de compensation de dose minimum, le manque de compensation de dose indiquant un manque, qui se produit par la non soumission à un rayonnement de la couche d'iso-énergie respective, en dépit d'une compensation correspondante par les couches d'iso-énergie exposées au rayonnement.

10. Système suivant la revendication 9,
**caractérisé**
**en ce que** le système ( 10 ) est conformé pour effectuer le procédé suivant l'une des revendications 1 à 8.

11. Installation d'exposition à un rayonnement de particules, comprenant un système ( 10 ) suivant la revendication 9 ou 10.

12. Produit de programme d'ordinateur, qui comprend un programme et qui peut être chargé directement dans une mémoire d'un dispositif de commande programmable d'une installation ( 20 ) d'exposition à un rayonnement particulaire, comprenant des moyens de programme pour effectuer tous les stades du procédé suivant l'une des revendications 1 à 8, lorsque le programme est exécuté dans le dispositif de commande de l'installation ( 20 ) d'exposition à un rayonnement particulaire.

13. Support de données déchiffrables électroniquement, ayant des informations de commande déchiffrables électroniquement, qui sont mises en mémoire et qui sont conformées de manière à ce que, lors de l'utilisation du support ( 29 ) de données dans un dispositif de commande d'une installation ( 20 ) d'exposition à un rayonnement particulaire, elles effectuent le procédé suivant l'une des revendications 1 à 8.
